(19) **Europäisches Patentamt**
**European Patent Office**
**Office européen des brevets**

(11) **EP 2 098 235 B1**

(12) **EUROPEAN PATENT SPECIFICATION**

(45) Date of publication and mention
of the grant of the patent:
**21.08.2013 Bulletin 2013/34**

(51) Int Cl.:
*A61K 31/445* (2006.01)  *A61K 9/70* (2006.01)
*A61K 47/02* (2006.01)  *A61K 47/14* (2006.01)
*A61K 47/32* (2006.01)  *A61P 25/28* (2006.01)
*A61K 47/22* (2006.01)  *A61K 47/20* (2006.01)

(21) Application number: **07849960.5**

(22) Date of filing: **30.11.2007**

(86) International application number:
**PCT/JP2007/073254**

(87) International publication number:
**WO 2008/066185 (05.06.2008 Gazette 2008/23)**

(54) **METHOD FOR PREVENTION OF DISCOLORATION WITH TIME OF DONEPEZIL-CONTAINING SKIN ADHESIVE PREPARATION**

VERFAHREN ZUR PRÄVENTION VON VERFÄRBUNG IM ZEITVERLAUF IN EINER DONEPEZILHALTIGEN HAUTKLEBEZUBEREITUNG

PROCÉDÉ DESTINÉ À PRÉVENIR LA DÉCOLORATION PROVOQUÉE PAR LE TEMPS D'UNE PRÉPARATION D'ADHÉSIF CUTANÉ CONTENANT DU DONÉPÉZIL

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **01.12.2006  JP 2006325104**
**01.12.2006  US 861965 P**
**07.06.2007  JP 2007152016**
**07.06.2007  JP 2007152047**

(43) Date of publication of application:
**09.09.2009 Bulletin 2009/37**

(73) Proprietors:
• **Nitto Denko Corporation**
**Ibaraki-shi**
**Osaka 567-8680 (JP)**
• **Eisai R&D Management Co., Ltd.**
**Tokyo 112-8088 (JP)**

(72) Inventors:
• **NISHI, Sumiyo**
**Ibaraki-shi**
**Osaka 567-8680 (JP)**
• **HANATANI, Akinori**
**Ibaraki-shi**
**Osaka 567-8680 (JP)**

• **SEKIYA, Junichi**
**Ibaraki-shi**
**Osaka 567-8680 (JP)**
• **TERASHI, Sachiko**
**Ibaraki-shi**
**Osaka 567-8680 (JP)**
• **AKEMI, Hitoshi**
**Ibaraki-shi**
**Osaka 567-8680 (JP)**
• **WASHIRO, Satoko**
**Ibaraki-shi**
**Osaka 567-8680 (JP)**

(74) Representative: **HOFFMANN EITLE**
**Patent- und Rechtsanwälte**
**Arabellastrasse 4**
**81925 München (DE)**

(56) References cited:
EP-A1- 1 086 706   EP-A1- 1 437 130
EP-A1- 1 541 177   WO-A1-2006/070930
WO-A1-2006/082728   JP-A- 2000 136 134
JP-A- 2007 217 328

• **MORIEARTY P L ET AL: "Transdermal patch delivery of acetylcholinesterase inhibitors", METHODS AND FINDINGS IN EXPERIMENTAL AND CLINICAL PHARMACOLOGY, PROUS, BARCELONA, ES, vol. 15, no. 6, 1 January 1993 (1993-01-01), pages 407-412, XP009120198, ISSN: 0379-0355**

**Description**

[Field of the Invention]

The present invention relates to a method for suppressing discoloration over time of adhesive preparations containing donepezil.

[Background of the Invention]

The basic drug donepezil has acetylcholine esterase inhibitory action, and is used as an anti-Alzheimer's dementia drug. Alzheimer's dementia patients are usually elderly, and elderly patients often have difficulty swallowing oral preparations. In addition, it may also be difficult in some cases to administer oral preparations to patients with advanced symptoms of Alzheimer's dementia. In these cases, percutaneous parenteral administration of donepezil is useful.

Adhesive preparations containing donepezil for percutaneous parenteral administration of donepezil are known, and are described, for example, in Japanese Patent Application Laid-open No. H11-315016 (Patent Document 1), WO 2003/032960 Pamphlet (Patent Document 2), and WO 2006/082728 Pamphlet (Patent Document 3) and the like. It is described in these Patent Documents 1 to 3 that antioxidants commonly used in orally administered preparations can be used, examples of which include tocopherol and ester derivatives thereof, ascorbic acid, ascorbyl stearate, nordihydroguaiaretic acid, dibutylhydroxytoluene (BHT) and butylhydroxyanisole. However, these Patent Documents 1 to 3 do not describe any examples of preparations in which antioxidants were actually blended or adequately verify the effectiveness of the antioxidants in the preparations.

In addition, Japanese Patent Application Laid-open No. 2000-136134 (Patent Document 4) describes that increases in donepezil-related substances can be inhibited by adding antioxidants such as sodium hydrogen sulfite, sodium sulfite, sodium pyrosulfite (sodium metabisulfite), cysteine, citric acid, edetate sodium (disodium ethylenediaminetetraacetate), ascorbic acid and erythorbic acid (isoascorbic acid) to an orally administered composition containing donepezil. However, Patent Document 4 proposes a liquid, syrup or other orally administered preparation, and does not suggest application of antioxidants to adhesive preparations. In addition, the effectiveness of antioxidants in adhesive preparations is not adequately verified.

On the other hand, adhesive preparations tend to be susceptible to the occurrence of changes in appearance over time due to the effects of oxygen in atmosphere, pH, temperature, humidity, light and the like. Even though these changes (discoloration) in the appearance of adhesive preparations, such as changes in hue, saturation or brightness, may be subtle in terms of the amounts thereof and not have any adverse effect whatsoever on the effectiveness of the adhesive preparation, they may perceived by a patient as indicating deterioration of quality, thereby resulting in the problem of causing the patient to refrain from their use. Problems with appearance stemming from discoloration of adhesive preparations in particular tend to be easily and keenly recognized by patients when an adhesive preparation appears white to light yellow (light brown) to the naked eye even at the time it is first used.

On the other hand, for example, Japanese Patent No. 3124069 (Patent Document 5), Japanese Patent Application Laid-open No. H11-047233 (Patent Document 6), Japanese Translation of PCT Application No. 2006-523637 (Patent Document 7) and Japanese Translation of PCT Application No. 2003-530422 (Patent Document 8) disclose technologies for suppressing discoloration in an adhesive preparation during long-term storage. However, these documents do not teach a method for suppressing discoloration over time of adhesive preparations containing donepezil.

Patent Document 1: Japanese Patent Application Laid-open No. H11-315016
Patent Document 2: WO 2003/032960
Patent Document 3: WO 2006/082728
Patent Document 4: Japanese Patent Application Laid-open No. 2000-136134
Patent Document 5: Japanese Patent No. 3124069
Patent Document 6: Japanese Patent Application Laid-open No. H11-047233
Patent Document 7: Japanese Translation of PCT Application No. 2006-523637
Patent Document 8: Japanese Translation of PCT Application No. 2003-530422

[Disclosure of the Invention]

[Problems To Be Solved by the Invention]

Under these circumstances, an object of the present invention is to suppress discoloration over time of adhesive preparations containing donepezil.

[Means for Solving the Problems]

As a result of exhaustive research, the inventors of the present invention found that discoloration over time of adhesive preparations containing donepezil can be suppressed by adding a specific stabilizer to the adhesive preparation containing donepezil, thereby leading to completion of the present invention.
Namely, the present invention is as described below.

(1) A method for suppressing discoloration over time of an adhesive preparation having a support and a pressure-sensitive adhesive layer, the pressure-sensitive adhesive layer containing a pressure-sensitive adhesive and donepezil, the method comprising:

including the pressure-sensitive adhesive, the donepezil and a stabilizer in the pressure-sensitive adhesive layer, wherein
the stabilizer is at least one species selected from the group consisting of ascorbic acid, a metal salt or an ester thereof, isoascorbic acid or a metal salt thereof, ethylenediamine tetraacetic acid or a metal salt thereof, 2-mercaptobenzimidazole, 3(2)-t-butyl-4-hydroxyanisole, 2,6-di-t-butyl-4-methylphenol, tetrakis[3-(3',5'-di-t-butyl-4'-hydroxyphenyl)propionic acid]pentaerythritol, (±)-α-tocopherol, (±)-α-tocopherol acetate, rutin, hypophosphorous acid, a metal metabisulfite salt and a metal salt of hydroxymethanesulfmic acid.

From the viewpoint of achieving adequate effects of the invention even at the initial stage following production of the adhesive preparation, in (1) above, the stabilizer is preferably at least one species selected from the group consisting of ethylenediamine tetraacetic acid or a metal salt thereof, 2-mercaptobenzimidazole, 3(2)-t-butyl-4-hydroxyanisole, 2,6-di-t-butyl-4-methylphenol, hypophosphorous acid, tetrakis[3-(3',5'-di-t-butyl-4'-hydroxyphenyl)propionic acid]pentaerythritol and (±)-α-tocopherol acetate.

(2) The method described in (1) above, wherein the stabilizer is at least one type selected from the following groups (a) to (e):

(a) combination of ascorbic acid, a metal salt or an ester thereof and at least one species selected from the group consisting of isoascorbic acid or a metal salt thereof, a metal metabisulfite salt, 2-mercaptobenzimidazole, 2,6-di-t-butyl-4-methylphenol, a metal salt of hydroxymethanesulfinic acid and rutin;
(b) combination of isoascorbic acid or a metal salt thereof and at least one species selected from the group consisting of 2-mercaptobenzimidazole, 2,6-di-t-butyl-4-methylphenol, a metal salt of hydroxymethanesulfmic acid and rutin;
(c) combination of 2-mercaptobenzimidazole and at least one species selected from the group consisting of 2,6-di-t-butyl-4-methylphenol, a metal salt of hydroxymethanesulfinic acid and rutin;
(d) combination of 2,6-di-t-butyl-4-methylphenol and a metal salt of hydroxymethanesulfinic acid; and
(e) combination of a metal salt of hydroxymethanesulfinic acid and rutin.

(3) The method described in (2) above, wherein the stabilizer is at least one type selected from the groups consisting of the following (a-1) to (c-1):

(a-1) combination of ascorbic acid, a metal salt or an ester thereof and at least one species selected from the group consisting of isoascorbic acid or a metal salt thereof, a metal metabisulfite salt, 2,6-di-t-butyl-4-methylphenol and a metal salt of hydroxymethanesulfinic acid;
(b-1) combination of isoascorbic acid or a metal salt thereof and at least one species selected from the group consisting of a metal salt of hydroxymethanesulfinic acid and 2-mercaptobenzimidazole; and
(c-1) combination of 2-mercaptobenzimidazole and at least one species selected from the group consisting of 2,6-di-t-butyl-4-methylphenol and a metal salt of hydroxymethanesulfinic acid.

From the viewpoint of achieving adequate effects of the invention even at the initial stage following production of the adhesive preparation, in (3) above, the stabilizer is preferably at least one type selected from the groups consisting of the following (b-2), (c-2) and (d-2):

(b-2) isoascorbic acid or a metal salt thereof and a metal salt of hydroxymethanesulfinic acid;
(c-2) 2-mercaptobenzimidazole and 2,6-di-t-butyl-4-methylphenol; and

(d-2) 2,6-di-t-butyl-4-methylphenol and a metal salt of hydroxymethanesulfmic acid.

(4) The method described in any one of (1) to (3) above, which comprises:

a step of: preparing a mixture comprising the pressure-sensitive adhesive, the donepezil,
and the stabilizer; and
a step of film-forming the pressure-sensitive adhesive layer by applying the mixture to a surface of the support.

[Advantageous Effects of the Invention]

According to the present invention, since discoloring over time can be suppressed for a long period of time, a highly stable donepezil-containing adhesive preparation can be realized, and the reliability of the preparation can be enhanced among physicians, patients and other users and handlers thereof.

[Best Mode for Carrying Out the Invention]

The following provides an explanation of the present invention in accordance with preferred embodiments thereof.
The method for suppressing discoloration over time of an adhesive preparation of the present invention comprises at least containing donepezil, a pressure-sensitive adhesive and a specific stabilizer in a pressure-sensitive adhesive layer in an adhesive preparation having a support and the pressure-sensitive adhesive layer formed on at least one side of the support.

Here, the term "donepezil" encompasses not only ($\pm$)-2-[(1-benzylpiperidin-4-yl)methyl]-5,6-dimethoxyindan-1-one (free form), but also pharmacologically acceptable salts and esters thereof.

In addition, the term "stabilizer" means a compound that has the action of being able to suppress discoloration over time (or changes in hue, saturation and brightness) in a pressure-sensitive adhesive layer containing donepezil and in a mixture of materials used to form a pressure-sensitive adhesive layer. In the present description, the degree of this discoloration over time is evaluated with the CIE1976 (L*a*b*) color system (L-star, a-star, b-star color system, JIS Z 8729).

In the present invention, the donepezil may be either donepezil (free form) or any of pharmaceutically acceptable salts or esters thereof. The pressure-sensitive adhesive layer preferably contains donepezil (free form) from the viewpoint of percutaneous absorbability

The adhesive preparation of the present invention can be used as an anti-Alzheimer's dementia drug. In addition, other possible applications include use against cerebrovascular dementia, prevention of migraine headaches and the like.

In the adhesive preparation of the present invention, the proportion of donepezil in the pressure-sensitive adhesive layer is preferably 1 to 30 wt% and more preferably 3 to 20 wt% based on the total weight of the pressure-sensitive adhesive layer. If the proportion is less than 1 wt%, a content effective for treatment cannot be expected to be released, while if the proportion exceeds 30 wt%, limitations arise on therapeutic effects while also resulting in the risk of being economically disadvantageous.

The stabilizer used in the present invention is a specific stabilizer that is at least one species selected from the group consisting of ascorbic acid, metal salts or esters thereof (preferably sodium salt or palmitic acid ester), isoascorbic acid or metal salts thereof (preferably sodium salt), ethylenediamine tetraacetic acid or metal salts thereof (preferably calcium disodium salt or tetrasodium salt), 2-mercaptobenzimidazole, 3(2)-t-butyl-4-hydroxyanisole, 2,6-di-t-butyl-4-methylphenol, tetrakis[3-(3',5'-di-t-butyl-4'-hydroxyphenyl)propionic acid]pentaerythritol, ($\pm$)-$\alpha$-tocopherol, ($\pm$)-$\alpha$-tocopherol acetate, rutin, hypophosphorous acid, metal metabisulfite salts (for example sodium salts) and metal salts of hydroxymethanesulfinic acid (preferably sodium salts).

Examples of the above-mentioned metals salts include sodium salts, potassium salts, calcium salts and magnesium salts. Examples of esters include palmitic acid esters, stearic acid esters and myristic acid esters.
In the present invention, in order to suppress discoloration over time of an adhesive preparation, the including of the pressure-sensitive adhesive, the donepezil and stabilizer of the above-mentioned method is preferably included in the production process of the adhesive preparation. Although the stabilizer may dissolve or disappear during production or storage of the adhesive preparation after the stabilizer has demonstrated the effects of the present invention, this case is also included in the scope of the present invention.

One species of the stabilizer may be used alone or two or more species thereof can be used in combination. In the case of using two or more species of the stabilizer in combination, examples of preferable aspects of combinations include the following (a) to (e):

(a) combination of ascorbic acid, metal salts or esters thereof (hereinafter generically referred to as "ascorbic acid derivatives") and at least one species selected from the group consisting of isoascorbic acid or metal salts thereof, metal metabisulfite salts, 2-mercaptobenzimidazole, 2,6-di-t-butyl-4-methylphenol, metal salts of hydroxymethanesulfinic acid and rutin;

(b) combination of isoascorbic acid or metal salts thereof and at least one species selected from the group consisting of 2-mercaptobenzimidazole, 2,6-di-t-butyl-4-methylphenol, metal salts of hydroxymethanesulfmic acid and rutin;

(c) combination of 2-mercaptobenzimidazole and at least one species selected from the group consisting of 2,6-di-t-butyl-4-methylphenol, metal salts of hydroxymethanesulfinic acid and rutin;

(d) combination of 2,6-di-t-butyl-4-methylphenol and metal salts of hydroxymethanesulfinic acid; and

(e) combination of metal salts of hydroxymethanesulfinic acid and rutin.

Among the combinations of stabilizers of (a) to (e) above, more preferable aspects thereof consist of the following (a-1) to (c-1). As a result of using these combinations (a-1) to (c-1), suppression effects on discoloration over time are demonstrated synergistically in comparison with the arithmetic mean value obtained when each of these stabilizers is used independently. From the viewpoint of achieving adequate effects of the invention even at the initial stage following production of the adhesive preparation, the stabilizer is more preferably at least one type selected from the group consisting of the following (b-2), (c-2) and (d-2):

(a-1) combination of ascorbic acid, metal salts or esters thereof and at least one species selected from the group consisting of isoascorbic acid or metal salts thereof, metal metabisulfite salts, 2,6-di-t-butyl-4-methylphenol and metal salts of hydroxymethanesulfinic acid;

(b-1) combination of isoascorbic acid or metal salts thereof and at least one species selected from the group consisting of metal salts of hydroxymethanesulfinic acid and 2-mercaptobenzimidazole;

(c-1) combination of 2-mercaptobenzimidazole and at least one species selected from the group consisting of 2,6-di-t-butyl-4-methylphenol and metal salts of hydroxymethanesulfinic acid;

(b-2) isoascorbic acid or metal salts thereof and metal salts of hydroxymethanesulfinic acid;

(c-2) 2-mercaptobenzimidazole and 2,6-di-t-butyl-4-methylphenol; and

(d-2) 2,6-di-t-butyl-4-methylphenol and metal salts of hydroxymethanesulfmic acid.

The stabilizer is required to be contained in the pressure-sensitive adhesive layer of the adhesive preparation. There are no particular limitations on the proportion of the weight of the stabilizer as far as it does not exert an adverse effect on the properties of the pressure-sensitive adhesive layer. Preferable examples of the upper limit value of the proportion of the stabilizer based on the total weight of the pressure-sensitive adhesive layer, in terms of the total content thereof, are such that a proportion in excess of 5 wt% results in the possibility of a decrease in the adhesiveness or other properties of the pressure-sensitive adhesive layer, while if the proportion is less than 0.0005 wt%, there is the possibility of adequate stabilizing effects being unable to be obtained. Thus, preferable examples of the upper limit value include 5 wt%, 3 wt%, 2 wt%, 1 wt%, 0.7 wt%, 0.5 wt% and 0.3 wt%, while preferable examples of the lower limit value are 0.0005 wt%, 0.001 wt%, 0.01 wt%, 0.02 wt%, 0.03 wt%, 0.05 wt%, 0.1 wt% and 0.2 wt%.

More specifically, the proportion of the total weight of the stabilizer based on the total weight of the pressure-sensitive adhesive layer is preferably 0.0005 to 5 wt%, more preferably 0.001 to 3 wt%, more preferably 0.01 to 1 wt%, more preferably 0.01 to 0.91 wt%, more preferably 0.01 to 0.7 wt%, more preferably 0.02 to 0.7 wt%, more preferably 0.02 to 0.5 wt% and most preferably 0.03 to 0.3 wt%.

In the aspects (a) to (e), (a-1) to (c-1) and (b-2) to (d-2) of the combinations of two species of compounds used for the stabilizers described above, the weight ratio of the two compounds is preferably 100:1 to 1:100, more preferably 10:1 to 1:100 and even more preferably 1:1 to 1:100.

In the adhesive preparation of the present invention, there are no particular limitations on the pressure-sensitive adhesive contained in the pressure-sensitive adhesive layer, and examples include acrylic pressure-sensitive adhesives; silicone rubber, polyisoprene rubber, polyisobutylene rubber, styrene-butadiene rubber, styrene-isoprene-styrene block copolymer rubber, styrene-butadiene-styrene block copolymer rubber and other rubber pressure-sensitive adhesives; silicone pressure-sensitive adhesives; and polyvinyl alcohol, polyvinyl alkyl ether, polyvinyl acetate and other vinyl polymer pressure-sensitive adhesives.

Since many rubber pressure-sensitive adhesives often do not have highly reactive functional groups, the donepezil contained therein is comparatively stable and the possibility of coloring in adhesives is comparatively low. Examples of such rubber pressure-sensitive adhesives include polyisobutylene and styrene-diene-styrene block copolymers (such as styrene-butadiene-styrene block copolymer (SBS) and styrene-isoprene-styrene block copolymer (SIS)), and one species may be used or a mixture of two or more species thereof may be used.

Depending on the type and the proportion of the copolymerized monomers, although acrylic pressure-sensitive adhesives have a comparatively high degree of freedom in terms of being able to control adhesive properties and the degree of drug solubility and the like, conversely their polymer chains may contain functional groups that are reactive with donepezil, and since residual monomers and polymerization initiators in the pressure-sensitive adhesive may also be reactive, there is concern over discoloration of the adhesive preparation. Thus, the present invention is carried out particularly advantageously in an adhesive preparation using an acrylic pressure-sensitive adhesive.

Examples of an acrylic pressure-sensitive adhesive in the present invention include acrylic pressure-sensitive adhesives containing a (meth)acrylic acid alkyl ester, and are preferably acrylic pressure-sensitive adhesives having a (meth)acrylic acid alkyl ester as a principal component (principal constituent unit) thereof. A copolymer of a (meth)acrylic acid alkyl ester (first monomer component) as the principal component with a vinyl monomer having functional groups capable of contributing to a crosslinking reaction (second monomer component), or a copolymer of these copolymerized with yet another monomer (third monomer component), is particularly preferable from the viewpoint of ease of crosslinking, pressure-sensitive adhesiveness with human skin, ability to manipulate drug dissolution and the like.

Preferable examples of the (meth)acrylic acid alkyl ester (first monomer component) include (meth)acrylic acid alkyl esters wherein the alkyl group is a linear, branched or cyclic alkyl group having 1 to 18 carbon atoms (such as methyl, ethyl, propyl, butyl, pentyl, hexyl, cyclohexyl, heptyl, octyl, 2-ethylhexyl, nonyl, decyl, undecyl, dodecyl or tridecyl), and (meth)acrylic acid alkyl esters wherein the alkyl group is a linear, branched or cyclic alkyl group having 4 to 18 carbon atoms (such as butyl, pentyl, hexyl, cyclohexyl, heptyl, octyl, 2-ethylhexyl, nonyl, decyl, undecyl, dodecyl or tridecyl). Moreover, since the use of a monomer component that lowers the glass transition temperature of the polymer is more preferable for imparting pressure-sensitive adhesiveness at normal temperatures, a (meth)acrylic acid alkyl ester wherein the alkyl group is a linear, branched or cyclic alkyl group having 4 to 8 carbon atoms (such as butyl, pentyl, hexyl, cyclohexyl, heptyl, octyl or 2-ethylhexyl, preferably butyl, 2-ethylhexyl or cyclohexyl and particularly preferably 2-ethylhexyl) is more preferable. More specifically, butyl acrylate, 2-ethylhexyl acrylate, 2-ethylhexyl methacrylate, cyclohexyl acrylate, cyclohexyl methacrylate or the like is preferable, and among these, 2-ethylhexyl acrylate is most preferable. One of these (meth)acrylic acid alkyl esters (first monomer component) may be used, or two or more may be used in combination.

In the vinyl monomer (second monomer component) having functional groups capable of contributing to the crosslinking reaction, examples of functional groups capable of contributing to the crosslinking reaction include hydroxyl groups, carboxyl groups and vinyl groups, and hydroxyl groups and carboxyl groups are preferable. Specific examples of this monomer (second monomer component) include hydroxyethyl (meth)acrylate esters, hydroxypropyl (meth)acrylate esters, (meth)acrylic acid, itaconic acid, maleic acid, maleic anhydride, mesaconic acid, citraconic acid and glutaconic acid and the like. Among these, acrylic acid, methacrylic acid and a hydroxyethyl acrylate ester (particularly 2-hydroxyethyl acrylate) are preferable from the viewpoint of availability, and acrylic acid is most preferable. One of these monomers (second monomer component) may be used, or two or more may be used in combination.

The above-mentioned other monomer (third monomer component) is used primarily to adjust the cohesiveness of the pressure-sensitive adhesive layer and to adjust the solubility or release properties of the donepezil and the like. Examples of this monomer (third monomer component) include vinyl acetate, vinyl propionate and other vinyl esters; methyl vinyl ether, ethyl vinyl ether and other vinyl ethers; N-vinyl-2-pyrrolidone, N-vinyl caprolactam and other vinyl amides; methoxyethyl (meth)acrylate ester, ethoxyethyl (meth)acrylate ester, tetrahydrofurfuryl (meth)acrylate ester and other alkoxy (meth)acrylate esters; hydroxypropyl (meth)acrylate, $\alpha$-hydroxymethyl acrylate and other hydroxyl group-containing monomers (which do not provide crosslinking sites because they are used as the third monomer component); (meth)acrylamide, dimethyl (meth)acrylamide, N-butyl (meth)acrylamide, N-methylol (meth)acrylamide and other (meth) acrylic acid derivatives having amide groups; aminoethyl (meth)acrylate ester, dimethylaminoethyl (meth)acrylate ester, t-butylaminoethyl (meth)acrylate ester and other aminoalkyl (meth)acrylate esters; methoxyethylene glycol (meth)acrylate ester, methoxydiethylene glycol (meth)acrylate ester, methoxypolyethylene glycol (meth)acrylate ester, methoxypolypropylene glycol (meth)acrylate ester and other alkoxyalkylene glycol (meth)acrylate esters; (meth)acrylonitrile; styrene sulfonic acid, allyl sulfonic acid, sulfopropyl (meth)acrylate, (meth)acryloyl oxynaphthalene sulfonic acid, acrylamide methyl sulfonic acid and other monomers having sulfonic acid; and vinyl piperidone, vinyl pyrimidine, vinyl piperazine, vinyl pyrrole, vinyl imidazole, vinyl oxazole, vinyl morpholine and other vinyl group-containing monomers. Among these, a vinyl ester or vinyl amide is preferable, and vinyl acetate is preferable as a vinyl ester, while N-vinyl-2-pyrrolidone is preferable as a vinyl amide. One of these monomers (third monomer component) may be used or two or more may be used in combination.

When the acrylic pressure-sensitive adhesive is a copolymer of a (meth)acrylic acid alkyl ester (first monomer component) and a vinyl monomer having functional groups capable of contributing to a crosslinking reaction (second monomer component), the (meth)acrylic acid alkyl ester and the vinyl monomer having functional groups capable of contributing to a crosslinking reaction are blended and copolymerized preferably at a weight ratio of 99 to 85 parts of (meth)acrylic acid alkyl ester per 1 to 15 parts of vinyl monomer having functional groups capable of contributing to a crosslinking reaction, and more preferably at a weight ratio of 99 to 90 parts per 1 to 10 parts.

In addition, when the acrylic pressure-sensitive adhesive is a copolymer of a (meth)acrylic acid alkyl ester (first monomer component), a vinyl monomer having functional groups capable of contributing to a crosslinking reaction (second monomer component) and another monomer (third monomer component), the (meth)acrylic acid alkyl ester, vinyl monomer having functional groups capable of contributing to a crosslinking reaction and other monomer are blended and copolymerized preferably at a weight ratio of 40 to 94 parts of (meth)acrylic acid alkyl ester per 1 to 15 parts of vinyl monomer having functional groups capable of being involved in a crosslinking reaction and 5 to 50 parts of other monomer, and more preferably at a weight ratio of 50 to 89 parts per 1 to 10 parts and 10 to 40 parts, respectively.

Although there are no particular limitations on the polymerization reaction as far as it is carried out with a known method, as an example thereof, a polymerization initiator (such as benzoyl peroxide, azobisisobutyronitrile or the like) is added to the above-mentioned monomers followed by reacting for 5 to 48 hours at 50 to 70°C in a solvent (such as ethyl acetate).

Particularly preferable examples of acrylic pressure-sensitive adhesives in the present invention include 2-ethylhexyl acrylate ester/acrylic acid/N-vinyl-2-pyrrolidone copolymer, 2-ethylhexyl acrylate ester/2-hydroxyethyl acrylate ester/vinyl acetate copolymer and 2-ethylhexyl acrylate ester/acrylic acid copolymer and the like, while a more preferable example is 2-ethylhexyl acrylate ester/acrylic acid/N-vinyl-2-pyrrolidone copolymer.

In addition, although varying according to the copolymer composition, the glass transition temperature of the acrylic pressure-sensitive adhesive in the present invention is normally preferably -100 to -10°C and more preferably -90 to -20°C from the viewpoint of adhesiveness of the adhesive preparation.

In the adhesive preparation of the present invention, a liquid component can be included in the pressure-sensitive adhesive layer from the viewpoint of imparting softness to the pressure-sensitive adhesive layer and reducing pain and skin irritation caused by skin adhesiveness when the adhesive preparation is peeled off the skin. An organic liquid component is preferable from the viewpoint of compatibility with the pressure-sensitive adhesive layer. In an adhesive preparation of the present invention containing an organic liquid component, there is the possibility of the coloring in the adhesive preparation due to a chemical reaction with the donepezil and the like depending on the type of organic liquid component. Thus, the present invention is carried out particularly advantageously in the case of an adhesive preparation containing an organic liquid component in terms of being able to efficiently inhibit this decrease in stability.

Although the organic liquid component can be used without any particular limitations as far as it is a liquid at room temperature, exhibits a plasticizing action and is compatible with the pressure-sensitive adhesive polymers composing the pressure-sensitive adhesive, that which improves the percutaneous absorbability and storage stability of donepezil is preferable. In addition, the organic liquid component can also be blended for the purpose of further enhancing the solubility of donepezil in the pressure-sensitive adhesive and the like. Examples of such organic liquid components include fatty acid alkyl esters (such as esters of lower monovalent alcohols having 1 to 4 carbon atoms and saturated or unsaturated fatty acids having 12 to 16 carbon atoms); saturated or unsaturated fatty acids having 8 to 10 carbon atoms (such as caprylic acid (octanoic acid, C8), pelargonic acid (nonanoic acid, C9), capric acid (decanoic acid, C10) or lauric acid (C12)); ethylene glycol, diethylene glycol, triethylene glycol, polyethylene glycol, propylene glycol, polypropylene glycol and other glycols; olive oil, castor oil, squalene, lanoline and other oils and fats; ethyl acetate, ethyl alcohol, dimethyl decyl sulfoxide, decyl methyl sulfoxide, dimethyl sulfoxide, dimethyl formamide, dimethyl acetamide, dimethyl lauryl amide, dodecyl pyrrolidone, isosorbitol, oleyl alcohol and other organic solvents; liquid surfactants; diisopropyl adipate, phthalic acid esters, diethyl sebacate and other plasticizers; and liquid paraffin and other hydrocarbons. In addition, other examples include ethoxylated stearyl alcohol, glycerin esters (those liquid at room temperature), isotridecyl myristate, N-methylpyrrolidone, ethyl oleate, oleic acid, diisopropyl adipate, octyl palmitate, 1,3-propanediol and glycerin. Among these, a fatty acid alkyl ester, saturated fatty acid, hydrocarbon or organic solvent is preferable from the viewpoint of stability of the preparation and the like, and a fatty acid alkyl ester is more preferable. One of these liquid organic components may be used alone or two or more may be used in combination.

In addition, in the case of using an acrylic pressure-sensitive adhesive for the pressure-sensitive adhesive, the organic liquid component is preferably a fatty acid alkyl ester from the viewpoint of compatibility and the like with the acrylic pressure-sensitive adhesive, and is more preferably an ester of a lower monovalent alcohol having 1 to 4 carbon atoms and a saturated or unsaturated fatty acid having 12 to 16 carbon atoms. Here, the saturated or unsaturated fatty acid having 12 to 16 carbon atoms is preferably a saturated fatty acid, while the lower monovalent alcohol having 1 to 4 carbon atoms may be either linear or branched. Preferable examples of fatty acids having 12 to 16 carbon atoms include lauric acid (C12), myristic acid (C14) and palmitic acid (C16) and the like, while preferable examples of lower monovalent alcohols having 1 to 4 carbon atoms include isopropyl alcohol, ethyl alcohol, methyl alcohol and propyl alcohol and the like. Specific examples of particularly preferable fatty acid alkyl esters include isopropyl myristate, ethyl laurate and isopropyl palmitate and the like.

Furthermore, in the case of using a fatty acid alkyl ester, a fatty acid having 8 to 10 carbon atoms and/or glycerin may be used in combination with the fatty acid alkyl ester from the viewpoint of improving the percutaneous absorbability of the donepezil.

The content of the organic liquid component blended in the present invention is preferably 10 to 160 parts by weight and more preferably 40 to 150 parts by weight based on 100 parts by weight of the pressure-sensitive adhesive. If the content is less than 10 parts by weight, favorable softness or the effect of reducing skin irritation may not be adequately obtained due to inadequate plasticization of the pressure-sensitive adhesive layer, while if the content exceeds 160 parts by weight, the organic liquid component may not be retained in the pressure-sensitive adhesive even by the cohesive force of the pressure-sensitive adhesive, thereby resulting in the adhesive force being weakened due to blooming on the surface of the pressure-sensitive adhesive layer, and increasing the possibility of the preparation detaching from the skin surface during use.

In the adhesive preparation of the present invention, the pressure-sensitive adhesive layer can be crosslinked by for example, a known chemical crosslinking process (crosslinking process using a crosslinking agent and the like) or physical crosslinking process (crosslinking process by exposing to ultraviolet rays or gamma (γ) rays or other electron rays and the like) as previously described, this crosslinking process may be that commonly used in the technical field. In the adhesive preparation of the present invention, the coloring in the adhesive preparation may occur depending on the chemical or physical crosslinking process used. Thus, the present invention is carried out particularly advantageously in an adhesive preparation in which the pressure-sensitive adhesive layer has been crosslinked. Furthermore, a chemical crosslinking process using a crosslinking agent is preferable from the viewpoint of being less likely to adversely affect the donepezil.

In the case of carrying out a chemical crosslinking process using a crosslinking agent, there are no particular limitations on the crosslinking agent so far as crosslink formation by such a crosslinking agent is not inhibited in the presence of donepezil, and examples include peroxides (such as benzoyl peroxide (BPO) and the like), metal oxides (such as magnesium metasilicate aluminate and the like), polyfunctional isocyanate compounds, organic metal compounds (such as zirconium and zinc alaninate , zinc acetate, glycine ammonium zinc or titanium compounds), metal alcoholates (such as tetraethyl titanate, tetraisopropyl titanate, aluminum isopropylate or aluminum sec-butyrate) and metal chelate compounds (such as dipropoxy bis(acetylacetonate) titanium, tetraoctylene glycol titanium, aluminum isopropylate, ethylacetoacetate aluminum diisopropylate, aluminum tris(ethylacetoacetate) or aluminum tris(acetylacetonate)). Among these, a peroxide, metal oxide, organic metal compound, metal alcoholate or metal chelate compound is preferable, and a metal alcoholate or metal chelate compound is more preferable from the viewpoint of efficiently forming crosslinks in the presence of donepezil, while a metal chelate compound is most preferable from the viewpoint of easily obtaining crosslinked structures with a suitable crosslinking density. In addition, among the metal chelate compounds, ethylacetoacetate aluminum diisopropylate is particularly preferable. One of these crosslinking agents may be used or two or more may be used in combination.

Although varying according to the type of crosslinking agent and pressure-sensitive adhesive, the content of the crosslinking agent blended is typically 0.1 to 0.6 parts by weight and preferably 0.15 to 0.5 parts by weight based on 100 parts by weight of the pressure-sensitive adhesive. If the content is less than 0.1 parts by weight, the crosslinking sites are too few to impart adequate cohesiveness to the pressure-sensitive adhesive layer, resulting in the risk of adhesive residue and strong skin irritation due to cohesive failure during peeling, while if the content exceeds 0:6 parts by weight, although cohesiveness is large, there are cases in which adequate adhesiveness may be unable to be obtained. In addition, there is also the risk of skin irritation caused by residual unreacted crosslinking agent.

Chemical crosslinking treatment can be carried out by, for example, adding the crosslinking agent followed by going through a step consisting of heating and storing at or above the crosslinking reaction temperature, or in other words a curing step. The heating temperature at this time is suitably selected according to the type of crosslinking agent, it is preferably 60 to 90°C and more preferably 60 to 80°C. The heating time is preferably 12 to 96 hours and more preferably 24 to 72 hours.

In the adhesive preparation of the present invention, a metal chloride can be contained together with donepezil in the crosslinked pressure-sensitive adhesive layer. Containing a metal chloride in the pressure-sensitive adhesive layer reduces the decrease in cohesiveness of the pressure-sensitive adhesive layer and the adhesive preparation is attached to human skin, and makes it less likely for cohesive failure to occur when the pressure-sensitive adhesive layer is peeled off.

There are no particular limitations on the metal chloride, and examples thereof include a chloride of an alkaline metal such as sodium or potassium; a chloride of an alkaline earth metal such as calcium or magnesium; aluminum chloride, stannous chloride and ferric chloride. From the viewpoint of superior stability and ability to inhibit decreases in cohesiveness of the pressure-sensitive adhesive layer, sodium chloride, calcium chloride, aluminum chloride, stannous chloride or ferric chloride is preferable, sodium chloride or calcium chloride is more preferable, and sodium chloride is particularly preferable. Any of these may be used alone or two or more may be used in combination. The content of the metal chloride blended is preferably 0.1 to 20 parts by weight, more preferably 1 to 15 parts by weight and most preferably 3 to 10 parts by weight based on 100 parts by weight of the pressure-sensitive adhesive. If the content is less than 0.1 parts by weight, the effect of inhibiting decreases in cohesiveness of the pressure-sensitive adhesive layer may be inadequate, while conversely if the content exceeds 20 parts by weight, although the inhibitory effect is demonstrated, the appearance of the preparation may be impaired due to the metal chloride not being uniformly dispersed in the pressure-sensitive adhesive (pressure-sensitive adhesive polymer).

In the present invention, the metal chloride may be that produced by neutralizing donepezil hydrochloride with an inorganic base containing a metal in the process of forming the pressure-sensitive adhesive layer. Examples of such inorganic bases containing metals include sodium hydroxide, potassium hydroxide, calcium hydroxide, magnesium hydroxide, sodium hydrogencarbonate, potassium hydrogencarbonate, sodium carbonate, potassium carbonate and other inorganic bases of alkaline metals or alkaline earth metals, a hydroxide of an alkaline metal or alkaline earth metal is preferable from the viewpoint of less likelihood of the formation of by-products, sodium hydroxide, calcium hydroxide and magnesium hydroxide are more preferable, and sodium hydroxide is particularly preferable.

In the adhesive preparation of the present invention, the thickness of the pressure-sensitive adhesive layer is preferably 20 to 300 $\mu$m, more preferably 30 to 300 $\mu$m and most preferably 50 to 300 $\mu$m. If the thickness of the pressure-sensitive adhesive layer is less than 20 $\mu$m, there is the risk of it being difficult to obtain adequate adhesive force or contain an effective content of donepezil, while if the thickness exceeds 300 $\mu$m, there is the risk of coating being difficult.

The adhesive preparation of the present invention has a support and a pressure-sensitive adhesive layer, and is preferably provided with a release liner. Namely, the adhesive preparation of the present invention has a structure wherein the pressure-sensitive adhesive layer described above is laminated on at least one side of the support, and the adhesive surface of the pressure-sensitive adhesive layer (the surface opposite the side on which the pressure-sensitive adhesive layer is laminated on the support) is preferably protected by being covered with a release liner until immediately before use. In addition, the adhesive preparation of the present invention may also be in the form of a roll without using a release liner by coating a silicone, fluorine or wax backing agent and the like onto the support.

Although there are no particular limitations on the support, that in which the proportion of donepezil in the pressure-sensitive adhesive layer does not decrease as a result of being lost from the back of the support by passing there through (namely, a material impermeable to donepezil) is preferable. And as will be described below, in the case of an aspect in which an organic liquid component is contained in the pressure-sensitive adhesive layer, a support in which the proportions of donepezil and organic liquid component do not decrease as a result of being lost from the back of the support by passing there through (namely, a material impermeable to the organic liquid component and the donepezil) is preferable.

Specific examples include polyester (such as polyethylene terephthalate (PET)), nylon, polyvinyl chloride, polyethylene, polypropylene, ethylene-vinyl acetate copolymer, polytetrafluoroethylene, ionomer resins and other single films, metal foil, and laminate films obtained by laminating two or more such films. Among these, the support is preferably that in which a laminate film is obtained by laminating a nonporous film consisting of one of the aforementioned materials with a porous film as described below in order to improve the adhesiveness (anchoring properties) of the support with the pressure-sensitive adhesive layer, and the pressure-sensitive adhesive layer is formed on the side of the porous film.

There are no particular limitations on the porous film as far as it improves anchoring properties with the pressure-sensitive adhesive layer, and examples include paper, woven fabrics, nonwoven fabrics (such as polyester (including polyethylene terephthalate (PET)) nonwoven fabric)), and films obtained by mechanical perforation of the above-mentioned films (such as polyester, nylon, Saran (product name), polyethylene, polypropylene, ethylene-vinyl acetate copolymer, polyvinyl chloride, ethylene-ethyl acrylate copolymer, polytetrafluoroethylene, metal foil, polyethylene terephthalate and other single films and laminate films obtained by laminating one or two or more such films), while paper, woven fabrics and nonwoven fabrics (such as polyester nonwoven fabric or polyethylene terephthalate nonwoven fabric) are preferable from the viewpoint of flexibility of the support. In consideration of improvement of anchoring properties and flexibility of the pressure-sensitive adhesive layer, the thickness of the porous film is normally 10 to 500 $\mu$m, and in the case of a thin adhesive preparation such as a plaster or adhesive tape, the thickness is normally about 1 to 200 $\mu$m. In the case of woven fabrics or nonwoven fabrics, the basis weight is preferably 5 to 30 g/m$^2$ in terms of improving anchoring properties.

There are no particular limitations on the thickness of the support for the adhesive preparation of the present invention, and is preferably 2 to 200 $\mu$m and more preferably 10 to 50 $\mu$m. If the thickness of the support is less than 2 $\mu$m, handling ease such as self-supporting properties tend to decrease, while if the thickness exceeds 200 $\mu$m, the support may feel unpleasant (stiff) and compliance with the skin tends to decrease.

There are no particular limitations on the release liner, and a known release liner can be used. Specific examples of the release liner include a release liner in which a release agent layer composed of a release agent is formed on the surface of a release liner base, a plastic film which itself has good release properties, and a release liner of a configuration in which a release layer, consisting of the plastic film material with good release properties, is formed on the surface of a release liner base. The release surface of the release liner may be on only one side of the base or on both sides.

There are no particular limitations on the release agent in this release liner, examples of which include long-chain alkyl group-containing polymers, silicone polymers (silicon release agents), fluorine polymers (fluorine release agents) and other release agents. Examples of the base for the release liner include polyethylene terephthalate (PET) film, polyimide film, polypropylene film, polyethylene film, polycarbonate film, polyester (other than PET) film or other plastic film; metal-deposited plastic films obtained by depositing a metal on one of these films; Japanese paper, regular paper, kraft paper, glassine paper, fine paper other paper; nonwoven fabric, fabric or other fibrous material; and metal foil.

In addition, examples of plastic films that themselves have good release properties include polyethylene (such a low-density polyethylene or linear low-density polyethylene), polypropylene, ethylene-propylene copolymer and other ethylene-$\alpha$-olefin copolymers (block copolymers or random copolymers) as well as polyolefin films formed from polyolefin resins consisting of mixtures thereof, and Teflon (registered trademark) films.

Furthermore, the release layer formed on the surface of the above-mentioned release liner base can be formed by laminating or coating the material of the above-mentioned plastic film with good release properties on the above-mentioned release liner base.

There are no particular limitations on the thickness (total thickness) of the release liner, and is normally 200 $\mu$m or less and preferably 25 to 100 $\mu$m.

In the present invention, although there are no particular limitations on the method for preparing the adhesive preparation, in one embodiment thereof, the adhesive preparation of the present invention is manufactured by film-forming of a mixture containing at least a pressure-sensitive adhesive, donepezil and a stabilizer to form the pressure-sensitive adhesive layer containing donepezil. Namely, the present invention also relates to a method for manufactured an adhesive preparation containing donepezil, which comprises forming a pressure-sensitive adhesive layer containing donepezil on at least one side of a support by film-forming of a mixture containing a pressure-sensitive adhesive, donepezil and the above-mentioned stabilizer thereon. Moreover, the present invention relates to a method for suppressing discoloration over time of an adhesive preparation containing donepezil, which comprises including a specific stabilizer together with donepezil in the presence of a pressure-sensitive adhesive.

In this embodiment, since the above-mentioned specific stabilizer is blended with the donepezil in the mixture for forming the pressure-sensitive adhesive layer, in addition to stabilizing the preparation from the time the mixture is prepared for forming the pressure-sensitive adhesive layer, discoloration over time of the donepezil-containing adhesive preparation is suppressed.

A specific example of a method consists of dissolving or dispersing a pressure-sensitive adhesive, donepezil and the above-mentioned stabilizer and the like in a solvent and mixing, coating the resulting solution or dispersion on at least one side of a support, and drying and depositing as a film to form the pressure-sensitive adhesive layer on the surface of the support followed by providing a release liner. Alternatively, for example, the above-mentioned solution or dispersion can be applied to at least one side of a protective release liner and then dried and deposited as a film to form the pressure-sensitive adhesive layer on the surface of the release liner, followed by adhering the support to the pressure-sensitive adhesive layer to prepare the adhesive preparation.

Examples of the solvent for dissolving and/or dispersing the pressure-sensitive adhesive and the like include ethyl acetate, toluene, hexane, 2-propanol, methanol, ethanol and water. In addition, these can also be used to adjust the viscosity after adding the crosslinking agent.

Furthermore, in the case the pressure-sensitive adhesive layer is to be crosslinked, the crosslinking agent is preferably added to the above-mentioned solution or dispersion as a chemical crosslinking treatment. In addition, in the case the pressure-sensitive adhesive layer is to be crosslinked, the pressure-sensitive adhesive layer is preferably stored (aging treatment (aging step)) to promote crosslinking in the pressure-sensitive adhesive layer after film formation. The aging treatment (aging step) is normally carried out after the solution or dispersion has been coated and dried to form a film (after formation of the pressure-sensitive adhesive layer) by allowing the resulting pressure-sensitive adhesive layer to stand for about 12 to 96 hours (preferably about 24 to 72 hours) while heating at 60 to 90°C (preferably 60 to 80°C). During this time, due to the presence of the above-mentioned stabilizer, coloring in the preparation is suppressed.

There are no particular limitations on the form of the adhesive preparation of the present invention, and may be in the form of a tape, sheet, matrix, reserver, controlled release film and the like. The stabilizing effect of the present invention is advantageous for tapes and sheets because these are susceptible to the effects of the environment and particularly oxygen.

Although varying according to the type and the content of pressure-sensitive adhesive and organic liquid component used as well as patient age, body weight, symptoms and the like, the dosage of the adhesive preparation of the present invention is preferably such that, in the case of an adult, an adhesive preparation containing 2 to 150 mg of donepezil or donepezil hydrochloride is normally administered to a 5 to 120 cm$^2$ patch of skin for about 1 to 7 days.

[Examples]

The following provides a more detailed explanation of the present invention through examples thereof. Furthermore, unless otherwise specified, "parts" in the description refers to "parts by weight".

Examples 1 to 15 and Comparative Examples 1 to 9

In an inert gas atmosphere, 75 parts of 2-ethylhexyl acrylate, 22 parts of N-vinyl-2-pyrrolidone, 3 parts of acrylic acid and 0.2 parts of azobisisobutyronitrile were solution-polymerized in ethyl acetate at 60°C to obtain an ethyl acetate solution of pressure-sensitive adhesive A (pressure-sensitive adhesive solid content: 28%).

Next, coating solutions, which contain the stabilizers shown in Table 2 below, were obtained by mixing the components shown in Table 1 below and adjusting the viscosity with ethyl acetate. These were coated onto polyethylene terephthalate (PET) release liners to a dried thickness of 60 μm, dried and laminated onto PET supports to obtain adhesive preparations followed by storing for 48 hours at 70°C to obtain the stored adhesive preparations of Examples 1 to 15 and Comparative Examples 1 to 9.

[Table 1]

| Pressure-sensitive adhesive A | 40 parts |
| Isopropyl myristate | 50 parts |
| Donepezil hydrochloride | 8.3 parts |
| Sodium hydroxide | 0.8 parts |
| Ethyl acetoacetate aluminum diisopropylate | 0.2 parts |
| Additives | 1.0 part |

Reference Example 1

The stored adhesive preparation (placebo preparation) of Reference Example 1 was obtained by treating in the same manner as Example 1 with the exception of not blending the donepezil hydrochloride and the two species of additives.

Reference Example 2

The stored adhesive preparation (control preparation) of Reference Example 2 was obtained by treating in the same manner as Example 1 with the exception of not blending the two species of additives.

(Measurement of Discoloration Over Time)

First, saturation of the adhesive preparation (placebo preparation) of Reference Example 1 was defined by the color values (L\*s,a\*s,b\*s) represented by the CIE1976 (L\*a\*b\*) color system (L-star, a-star, b-star color system, JIS Z 8729), after which the respective saturations of the adhesive preparations of Examples 1 to 15 and Comparative Examples 1 to 9 along with the adhesive preparation (control preparation) of Reference Example 2 were defined by the color values (L\*1,a\*1,b\*1) represented by the CIE1976 (L\*a\*b\*) color system (L-star, a-star, b-star color system, JIS Z 8729).

The color differences (ΔE\*ab1) between the color values (L\*1,a\*1,b\*1) of the adhesive preparations of Examples 1 to 15 and Comparative Examples 1 to 9 along with the adhesive preparation of Reference Example 2 (control preparation) and the color value (L\*s,a\*s,b\*s) of the adhesive preparation of Reference Example 1 (placebo preparation) as reference point were calculated according to formula 1 below. The results are shown in Table 2.

$$\Delta E^*ab1 = [(L^*1 - L^*s)^2 + (a^*1 - a^*s)^2 + (b^*1 - b^*s)^2]^{1/2} \qquad \text{(Formula 1)}$$

Next, the adhesive preparations of Examples 1 to 15 and Comparative Examples 1 to 9 along with the adhesive preparation of Reference Example 2 (control preparation) were stored for 8 to 11 months under conditions of a constant temperature of 23°C, followed by measuring the color values (L\*t,a\*t,b\*t) of the adhesive preparations of Examples 1 to 15 and Comparative Examples 1 to 9 along with the adhesive preparation of Reference Example 2 (control preparation) after long-term storage in the same manner as described above.

The color differences (ΔE\*ab2) between the color values (L\*t,a\*t,b\*t) of the adhesive preparations of Examples 1 to 15 and Comparative Examples 1 to 9 along with the adhesive preparation of Reference Example 2 (control preparation) after long-term storage and the color values (L\*1,a\*1,b\*1) as reference point of each of the adhesive preparations described above before storage were then calculated according to formula 2 below. The results are shown in Table 2.

$$\Delta E^*ab2 = [(L^*t - L^*1)^2 + (a^*t - a^*1)^2 + (b^*t - b^*1)^2]^{1/2} \qquad \text{(Formula 2)}$$

EP 2 098 235 B1

[Table 2]

| | Stabilizer | Color value after storing for 48 hours at 70°C (L*,a*,b*) | | | Color difference with Ref. Ex. 1 (placebo preparation) ΔE*ab1 | Color difference after long-term storage (L*,a*,b*) | | | Comparison before and after storage | | | | |
|---|---|---|---|---|---|---|---|---|---|---|---|---|---|
| | | L*1 (L*s) | a*1 (a*s) | b*1 (b*s) | | L*t | a*t | b*t | ΔL* | Δa* | Δb* | Color difference before and after storage ΔE*ab2 | Evaluation |
| Ref. Ex. 1 | Placebo preparation (no antioxidant, drug absent) | 96.4 | -5.5 | 8.2 | - | - | - | - | - | - | - | - | - |
| Ref. Ex. 2 | Control (no antioxidant, drug present) | 95.4 | -6.1 | 11.2 | 3.2 | 95.1 | -6.5 | 13.1 | -0.3 | -0.4 | 1.9 | 2.0 | - |
| Ex. 1 | D(-)-isoascorbic acid | 92.6 | -5.8 | 23.1 | 15.4 | 93.4 | -6.4 | 22.2 | 0.8 | -0.6 | -0.9 | 1.3 | ○ |
| Ex. 2 | Sodium isoascorbate monohydrate | 95.4 | -6.5 | 12.6 | 4.6 | 95.5 | -6.5 | 12.4 | 0.1 | 0.0 | -0.2 | 0.2 | ⊗ |
| Ex. 3 | Ethylenediamine-N,N, N'N'- calcium tetraacetate (II) disodium salt dihydrate | 95.9 | -5.8 | 9.5 | 1.4 | 95.9 | -6.3 | 10.8 | 0.0 | -0.5 | 1.3 | 1.4 | ○ |
| Ex.4 | 2-mercaptobenzimidazole | 96.1 | -5.6 | 8.5 | 0.4 | 96.2 | -5.8 | 8.9 | 0.1 | -0.2 | 0.4 | 0.5 | ⊗ |
| Ex. 5 | L(+)-ascorbic acid | 89.7 | -4.0 | 26.4 | 19.5 | 90.8 | -4.8 | 26.0 | 1.1 | -0.8 | -0.4 | 1.4 | ○ |
| Ex. 6 | L-ascorbyl palmitate | 93.1 | -6.2 | 20.5 | 12.8 | 93.2 | -6.4 | 20.2 | 0.1 | -0.2 | -0.3 | 0.4 | ⊗ |
| Ex. 7 | 3(2)-t-butyl-4-hydroxyanisol e | 95.3 | -6.0 | 9.7 | 1.9 | 95.1 | -6.0 | 10.3 | -0.2 | 0.0 | 0.6 | 0.6 | ⊗ |
| Ex. 8 | 2,6-di-t-butyl-4-methylphen ol | 96.1 | -5.6 | 8.3 | 0.3 | 96.2 | -5.7 | 8.4 | 0.1 | -0.1 | 0.1 | 0.2 | ⊗ |
| Ex. 9 | Hypophosphorous acid | 95.3 | -6.1 | 10.5 | 2.6 | 95.5 | -6.4 | 11.8 | 0.2 | -0.3 | 1.3 | 1.3 | ○ |
| Ex. 10 | Tetrakis[3-(3',5'-di-t-butyl- | 95.8 | -5.7 | 8.9 | 0.9 | 95.9 | -5.7 | 8.8 | 0.1 | 0.0 | -0.1 | 0.1 | ⊗ |

12

(continued)

| | Stabilizer | Color value after storing for 48 hours at 70°C (L*,a*,b*) | | | Color difference with Ref. Ex. 1 (placebo preparation) $\Delta E^*ab1$ | Color difference after long-term storage (L*,a*,b*) | | | Comparison before and after storage | | | | |
|---|---|---|---|---|---|---|---|---|---|---|---|---|---|
| | | $L^*1$ (L*s) | $a^*1$ (a*s) | $b^*1$ (b*s) | | $L^*t$ | $a^*t$ | $b^*t$ | $\Delta L^*$ | $\Delta a^*$ | $\Delta b^*$ | Color difference before and after storage $\Delta E^*ab2$ | Evaluation |
| | 4'-hydroxyphenyl) propionic acid] pentaerythritol | | | | | | | | | | | | |
| Ex. 11 | Sodium hydroxymethane-sulfinate dihydrate | 94.6 | -7.7 | 19.7 | 11.8 | 94.6 | -7.8 | 19.9 | 0.0 | -0.1 | 0.2 | 0.2 | ⊗ |
| Ex. 12 | Sodium metabisulfite | 91.6 | -5.8 | 21.7 | 14.3 | 91.6 | -6.2 | 23.6 | 0.0 | -0.4 | 1.9 | 1.9 | ○ |
| Ex. 13 | (±)-α-tocopherol | 92.4 | -8.2 | 14.8 | 8.2 | 91.9 | -7.2 | 15.0 | -0.5 | 1.0 | 0.2 | 1.1 | ○ |
| Ex. 14 | (±)-α-tocopherol acetate | 95.6 | -6.0 | 10.3 | 2.3 | 95.6 | -6.5 | 11.7 | 0.0 | -0.5 | 1.4 | 1.5 | ○ |
| Ex. 15 | Rutin | 91.7 | -11.4 | 70.5 | 62.8 | 91.7 | -11.3 | 72.3 | 0.0 | 0.1 | 1.8 | 1.8 | ○ |
| Comp. Ex. 1 | L-cysteine | 95.1 | -7.2 | 15.2 | 7.3 | 94.8 | -7.2 | 17.5 | -0.3 | 0.0 | 2.3 | 2.3 | × |
| Comp. Ex. 2 | 3-mercapto-1,2-propanediol | 93.8 | -7.0 | 18.8 | 11.0 | 93.6 | -7.2 | 20.9 | -0.2 | -0.2 | 2.1 | 2.1 | × |
| Comp. Ex. 3 | Propyl gallate | 94.7 | -6.4 | 12.3 | 4.5 | 94.3 | -6.9 | 14.6 | -0.4 | -0.5 | 2.3 | 2.4 | × |
| Comp. Ex. 4 | 1,3-butanediol | 95.4 | -6.1 | 11.0 | 3.0 | 95.4 | -6.6 | 13.1 | 0.0 | -0.5 | 2.1 | 2.2 | × |
| Comp. Ex. 5 | Sodium sulfite | 94.2 | -6.3 | 14.2 | 6.4 | 94.2 | -6.8 | 16.2 | 0.0 | -0.5 | 2.0 | 2.1 | × |
| Comp. Ex. 6 | Sodium thiosulfate | 95.1 | -5.9 | 11.9 | 3.9 | 94.4 | -6.5 | 15.2 | -0.7 | -0.6 | 3.3 | 3.4 | × |
| Comp. Ex. 7 | Quercetin dihydrate | 84.2 | 6.8 | 82.5 | 76.3 | 84.2 | 5.8 | 84.6 | 0.0 | -1.0 | 2.1 | 2.3 | × |

EP 2 098 235 B1

13

(continued)

| | Stabilizer | Color value after storing for 48 hours at 70°C (L*,a*,b*) | | | Color difference with Ref. Ex. 1 (placebo preparation) $\Delta E^*ab1$ | Color difference after long-term storage (L*,a*,b*) | | | Comparison before and after storage | | | | |
|---|---|---|---|---|---|---|---|---|---|---|---|---|---|
| | | L*1 (L*s) | a*1 (a*s) | b*1 (b*s) | | L*t | a*t | b*t | $\Delta L^*$ | $\Delta a^*$ | $\Delta b^*$ | Color differen ce before and after storage $\Delta E^*ab2$ | Eva luat ion |
| Comp. Ex. 8 | Hydroquinone | 90.9 | -3.0 | 15.3 | 9.3 | 89.0 | -1.6 | 17.2 | -1.9 | 1.4 | 1.9 | 3.0 | × |
| Comp. Ex. 9 | 1H-benzotriazole | 95.4 | -6.3 | 11.6 | 3.6 | 95.4 | -6.9 | 13.5 | 0.0 | -0.6 | 1.9 | 2.0 | × |

As is clear from Table 2, the adhesive preparations of Examples 1 to 15 exhibited lower values for color difference $\Delta E^*ab2$ before and after long-term storage as compared with Comparative Examples 1 to 9 and Reference Example 2. On the basis thereof, discoloration over time of the donepezil-containing adhesive preparations was confirmed to be suppressed in the adhesive preparations of Examples 1 to 15.

Examples 16 to 30

The stored adhesive preparations of Examples 16 to 30 were obtained by following the same procedure as Example 1 with the exception of changing the stabilizer to the two species of stabilizers (0.5 parts by weight each) shown in Table 3.

The color values ($L^*1,a^*1,b^*1$) of the stored adhesive preparations and the color values ($L^*t,a^*t,b^*t$) after long-term storage of the resulting Examples 16 to 30 were measured in the same manner as previously described, and each of the values for $\Delta E^*ab1$ and $\Delta E^*ab2$ were calculated with the above-mentioned formula 1 and formula 2, respectively. The results are shown in Table 3.

[Table 3]

| | Stabilizers | | Color value after storing for 48 hours at 70°C (L*, a*, b*) | | | Color difference with Ref. Ex. 1 (placebo preparation) ΔE*ab1 | Color difference after long-term storage (L*, a*, b*) | | | Comparison before and after storage | | | | Evaluation |
|---|---|---|---|---|---|---|---|---|---|---|---|---|---|---|
| | | | L*1 (L*s) | a*1 (a*s) | b*1 (b*s) | | L*t | a*t | b*t | ΔL* | Δa* | Δb* | Color difference before and after storage ΔE*ab2 | |
| Ref. Ex. 1 | Placebo preparation (no antioxidant, drug absent) | | 96.4 | -5.5 | 8.2 | - | - | - | - | - | - | - | - | - |
| Ref. Ex. 2 | Control (no antioxidant, drug present) | | 95.4 | -6.1 | 11.2 | 3.2 | 95.1 | -6.5 | 13.1 | -0.3 | -0.4 | 1.9 | 2.0 | - |
| Ex. 16 | L(+)-ascorbic acid | Sodium metabisulfite | 94.2 | -5.8 | 19.1 | 11.1 | 94.1 | -5.9 | 18.6 | -0.1 | -0.1 | -0.5 | 0.5 | ⊗ |
| Ex. 17 | L(+)-ascorbic acid | D(-)-isoascorbic acid | 92.5 | -5.7 | 24.1 | 16.4 | 92.9 | -6.1 | 23.4 | 0.4 | -0.4 | -0.7 | 0.9 | ⊗ |
| Ex. 18 | L(+)-ascorbic acid | 2-mercaptobenzimidazole | 90.9 | -4.0 | 24.8 | 17.6 | 91.4 | -4.6 | 23.8 | 0.5 | -0.6 | -1.0 | 1.3 | ○ |
| Ex. 19 | L(+)-ascorbic acid | 2,6-di-t-butyl-4-methylphenol | 93.6 | -5.8 | 18.6 | 10.8 | 93.8 | -6.0 | 18.3 | 0.2 | -0.2 | -0.3 | 0.4 | ⊗ |
| Ex. 20 | L(+)-ascorbic acid | Sodium hydroxymethane-sulfinate dihydrate | 95.8 | -6.7 | 12.4 | 4.4 | 95.7 | -6.8 | 12.5 | -0.1 | -0.1 | 0.1 | 0.2 | ⊗ |
| Ex. 21 | L(+)-ascorbic acid | Rutin | 92.8 | -13.1 | 52.9 | 45.5 | 93.1 | -14.0 | 54.2 | 0.3 | -0.9 | 1.3 | 1.6 | ○ |
| Ex. 22 | D(-)-isoascorbic acid | 2-mercaptobenzimidazole | 94.0 | -6.2 | 19.1 | 11.2 | 94.0 | -6.4 | 19.3 | 0.0 | -0.2 | 0.2 | 0.3 | ⊗ |

(continued)

| | Stabilizers | | Color value after storing for 48 hours at 70°C (L*, a*, b*) | | | Color difference with Ref. Ex.1 (placebo preparation) ΔE*ab1 | Color difference after long-term storage (L*, a*, b*) | | | Comparison before and after storage | | | | |
|---|---|---|---|---|---|---|---|---|---|---|---|---|---|---|
| | | | L*1 (L*s) | a*1 (a*s) | b*1 (b*s) | | L*t | a*t | b*t | ΔL* | Δa* | Δb* | Color difference before and after storage ΔE*ab2 | Evaluation |
| Ex. 23 | D(-)-isoascorbic acid | 2,6-di-t-butyl-4-methylphenol | 95.0 | -7.1 | 16.8 | 8.9 | 95.1 | -7.1 | 16.1 | 0.1 | 0.0 | -0.7 | 0.7 | ○ |
| Ex. 24 | D(-)-isoascorbic acid | Sodium hydroxymethane-sulfinate dihydrate | 96.1 | -6.5 | 11.0 | 3.0 | 96.1 | -6.4 | 10.6 | 0.0 | 0.1 | -0.4 | 0.4 | ⊗ |
| Ex. 25 | D(-)-isoascorbic acid | Rutin | 94.0 | -15.0 | 54.8 | 47.6 | 94.2 | -15.4 | 53.8 | 0.2 | -0.4 | -1.0 | 1.1 | ○ |
| Ex. 26 | 2-mercaptobenzimidazole | 2,6-di-t-butyl-4-methylphenol | 96.2 | -5.6 | 8.2 | 0.2 | 96.2 | -5.8 | 8.2 | 0.0 | -0.2 | 0.0 | 0.2 | ⊗ |
| Ex. 27 | 2-mercaptobenzimidazole | Sodium hydroxymethane-sulfinate dihydrate | 94.9 | -7.0 | 16.8 | 8.9 | 94.8 | -7.1 | 16.9 | -0.1 | -0.1 | 0.1 | 0.2 | ⊗ |
| Ex. 28 | 2-mercaptobenzimidazole | *Rutin* | 93.5 | -14.0 | 59.3 | 51.9 | 93.5 | -14.2 | 60.5 | 0.0 | -0.2 | 1.2 | 1.2 | ○ |
| Ex. 29 | 2,6-di-t-butyl-4-methylphenol | Sodium hydroxymethane-sulfinate dihydrate | 96.0 | -5.6 | 8.9 | 0.8 | 96.1 | -5.8 | 8.9 | 0.1 | -0.2 | 0.0 | 0.2 | ○ |
| Ex. 30 | Sodium hydroxymethane-sulfinate dihydrate | Rutin | 93.1 | -12.8 | 54.6 | 47.1 | 93.1 | -13.2 | 56.3 | 0.0 | -0.4 | 1.7 | 1.7 | ○ |

As is clear from Table 3, the adhesive preparations of Examples 16 to 30 exhibited lower values for color difference ΔE*ab2 before and after long-term storage as compared with Comparative Examples 1 to 9 and Reference Example 2. On the basis thereof, discoloration over time of the donepezil-containing adhesive preparations was confirmed to be suppressed.

In addition, in Examples 16, 17, 19, 20, 22, 24, 26 and 27, the value for color difference ΔE*ab2 before and after long-term storage was lower than the arithmetic mean of the suppression effects over time for each of the stabilizers used alone (values calculated based on the values for color difference ΔE*ab2 of Examples 1, 4, 5, 8, 11 and 12 consisting of 1.1, 1.3, 0.8, 34.5, 0.7, 0.6, 0.3 and 0.3, respectively). On the basis thereof, combining the stabilizers used in these examples was determined to result in the suppression effects on discoloration over time of the donepezil-containing adhesive preparations being demonstrated synergistically.

According to the present invention, since discoloration over time of a donepezil-containing adhesive preparation can be suppressed enabling a constant appearance to be maintained for a long period of time, and since the reliability of the preparation can be enhanced among physicians, patients and other users and handlers thereof, the present invention can be widely and effectively used as an anti-Alzheimer's dementia drug, anti-cerebrovascular dementia drug, prevention of migraine headaches and other pharmaceutical applications by parenteral administration, and particularly by percutaneous administration.

## Claims

1. method for suppressing discoloration over time of an adhesive preparation having a support and a pressure-sensitive adhesive layer, the pressure-sensitive adhesive layer containing a pressure-sensitive adhesive and donepezil, the method comprising:

   including the pressure-sensitive adhesive, the donepezil and a stabilizer in the pressure-sensitive adhesive layer, wherein
   the stabilizer is at least one species selected from the group consisting of ascorbic acid, a metal salt or an ester thereof, isoascorbic acid or a metal salt thereof, ethylenediamine tetraacetic acid or a metal salt thereof, 2-mercaptobenzimidazole, 3(2)-t-butyl-4-hydroxyanisole, 2,6-di-t-butyl-4-methylphenol, tetrakis[3-(3',5'-di-t-butyl-4'-hydroxyphenyl)propionic acid]pentaerythritol, ($\pm$)-$\alpha$-tocopherol, ($\pm$)-$\alpha$-tocopherol acetate, rutin, hypo-phosphorous acid, a metal metabisulfite salt and a metal salt of hydroxymethanesulfinic acid.

2. The method according to claim 1, wherein the stabilizer is at least one type selected from the following groups (a) to (e):

   (a) combination of ascorbic acid, a metal salt or an ester thereof and at least one species selected from the group consisting of isoascorbic acid or a metal salt thereof, a metal metabisulfite salt, 2-mercaptobenzimidazole, 2,6-di-t-butyl-4-methylphenol, a metal salt of hydroxymethanesulfinic acid and rutin;
   (b) combination of isoascorbic acid or a metal salt thereof and at least one species selected from the group consisting of 2-mercaptobenzimidazole, 2,6-di-t-butyl-4-methylphenol, a metal salt of hydroxymethanesulfinic acid and rutin;
   (c) combination of 2-mercaptobenzimidazole and at least one species selected from the group consisting of 2,6-di-t-butyl-4-methylphenol, a metal salt of hydroxymethanesulfinic acid and rutin;
   (d) combination of 2,6-di-t-butyl-4-methylphenol and a metal salt of hydroxymethanesulfinic acid; and
   (e) combination of a metal salt of hydroxymethanesulfinic acid and rutin.

3. The method according to claim 2, wherein the stabilizer is at least one type selected from the groups consisting of the following (a-1) to (c-1):

   (a-1) combination of ascorbic acid, a metal salt or an ester thereof and at least one species selected from the group consisting of isoascorbic acid or a metal salt thereof, a metal metabisulfite salt, 2,6-di-t-butyl-4-methylphenol and a metal salt of hydroxymethanesulfinic acid;
   (b-1) combination of isoascorbic acid or a metal salt thereof and at least one species selected from the group consisting of a metal salt of hydroxymethanesulfinic acid and 2-mercaptobenzimidazole; and
   (c-1) combination of 2-mercaptobenzimidazole and at least one species selected from the group consisting of 2,6-di-t-butyl-4-methylphenol and a metal salt of hydroxymethanesulfinic acid.

4. The method according to any one of claims 1 to 3, which comprises:

a step of preparing a mixture comprising the pressure-sensitive adhesive, the donepezil, and the stabilizer; and
a step of film-forming the pressure-sensitive adhesive layer by applying the mixture to a surface of the support.

**Patentansprüche**

1. Verfahren zur Unterdrückung einer zeitlichen Verfärbung einer Haftzubereitung, die einen Träger und eine druckempfindliche Haftschicht aufweist, wobei die druckempfindliche Haftschicht einen druckempfindlichen Klebstoff und Donepezil enthält; das Verfahren umfasst:

   Einschliessen des druckempfindlichen Klebstoffs, des Donezepils und eines Stabilisators in der druckempfindlichen Haftschicht, wobei
   der Stabilisator mindestens eine Art, ausgewählt aus der Gruppe bestehend aus Ascorbinsäure, einem Metallsalz oder einem Ester davon, Isoascorbinsäure oder einem Metallsalz davon, Ethylendiamintetraessigsäure oder einem Metallsalz davon, 2-Mercaptobenzimidazol, 3(2)-t-Butyl-4-hydroxyanisol, 2,6-Di-t-butyl-4-methylphenol, Tetrakis[3-(3',5'-di-t-butyl-4'-hydroxyphenyl)-propionsäure]pentaerythrit, (±)-α-Tocopherol, (±)-α-Tocopherolacetat, Rutin, hypophosphoriger Säure, einem Metallbisulfitsalz und einem Metallsalz von Hydroxymethansulfinsäure, ist.

2. Verfahren gemäss Anspruch 1, wobei der Stabilisator mindestens eine Art, ausgewählt aus den nachstehenden Gruppen (a) bis (e), ist:

   (a) eine Kombination aus Ascorbinsäure, einem Metallsalz oder einem Ester davon und mindestens einer Art, ausgewählt aus der Gruppe bestehend aus Isoascorbinsäure oder einem Metallsalz davon, einem Metallmetabisulfitsalz, 2-Mercaptobenzimidazol, 2,6-Di-t-butyl-4-methylphenol, einem Metallsalz von Hydroxymethansulfinsäure und Rutin;
   (b) eine Kombination aus Isoascorbinsäure oder einem Metallsalz davon und mindestens einer Art, ausgewählt aus der Gruppe bestehend aus 2-Mercaptobenzimidazol, 2,6-Di-t-butyl-4-methylphenol, einem Metallsalz von Hydroxymethansulfonsäure und Rutin;
   (c) eine Kombination aus 2-Mercaptobenzimidazol und mindestens einer Art, ausgewählt aus der Gruppe bestehend aus 2,6-Di-t-butyl-4-methylphenol, einem Metallsalz von Hydroxymethansulfinsäure und Rutin;
   (d) eine Kombination aus 2,6-Di-t-butyl-4-methylphenol und einem Metallsalz von Hydroxymethansulfinsäure; und
   (e) eine Kombination aus einem Metallsalz von Hydroxymethansulfinsäure und Rutin.

3. Verfahren gemäss Anspruch 2, wobei der Stabilisator mindestens eine Art, ausgewählt aus den Gruppen bestehend aus den nachstehenden (a-1) bis (c-1), ist:

   (a-1) eine Kombination aus Ascorbinsäure, einem Metallsalz oder einem Ester davon und mindestens einer Art, ausgewählt aus der Gruppe bestehend aus Isoascorbinsäure oder einem Metallsalz davon, einem Metallmetabilsulfitsalz, 2,6-Di-t-butyl-4-methylphenol und einem Metallsalz von Hydroxymethansulfinsäure;
   (b-1) eine Kombination aus Isoascorbinsäure oder einem Metallsalz davon und mindestens einer Art, ausgewählt aus der Gruppe bestehend aus einem Metallsalz von Hydroxymethansulfinsäure und 2-Mercaptobenzimidazol; und
   (c-1) eine Kombination aus 2-Mercaptobenzimidazol und mindestens einer Art, ausgewählt aus der Gruppe bestehend aus 2,6-Di-t-butyl-4-methylphenol und einem Metallsalz von Hydroxymethansulfinsäure.

4. Verfahren gemäss irgendeinem der Ansprüche 1 bis 3, umfassend:

   einen Schritt zur Herstellung einer Mischung, die das druckempfindliche Haftmittel, das Donepezil und den Stabilisator enthält; und
   einen Schritt zur Filmbildung der druckempfindlichen Haftschicht durch Aufbringen der Mischung an der Oberfläche des Trägers.

**Revendications**

1. Procédé pour supprimer la décoloration dans le temps d'une préparation adhésive ayant un support et une couche adhésive sensible à la pression, la couche adhésive sensible à la pression contenant un adhésif sensible à la pression et du donépézil, le procédé comprenant :

l'inclusion de l'adhésif sensible à la pression, du donépézil et d'un stabilisant dans la couche adhésive sensible à la pression, dans lequel

le stabilisant est au moins une espèce sélectionnée à partir du groupe constitué par l'acide ascorbique, un sel métallique ou un ester de celui-ci, l'acide isoascorbique ou un sel métallique de celui-ci, l'acide éthylène diamine tétra-acétique ou un sel métallique de celui-ci, le 2-mercaptobenzimidazole, le 3(2)-t-butyl-4-hydroxyanisole, le 2,6-di-t-butyl-4-méthylphénol, le tétrakis[3-(3',5'-di-t-butyl-4'-hydroxyphényl)acide propionique]pentaérythritol, le ($\pm$)-$\alpha$-tocophérol, l'acétate de ($\pm$)-$\alpha$-tocophérol, la rutine, l'acide hypophosphoreux, un sel métallique de métabisulfite et un sel métallique d'acide hydroxyméthanesulfinique.

2. Procédé selon la revendication 1, dans lequel le stabilisant est au moins un type sélectionné à partir des groupes suivants (a) à (e) :

(a) combinaison d'acide ascorbique, un sel métallique ou un ester de celui-ci et d'au moins une espèce sélectionnée à partir du groupe constitué par l'acide isoascorbique ou un sel métallique de celui-ci, un sel de métabisulfite métallique, le 2-mercaptobenzimidazole, le 2,6-di-t-butyl-4-méthylphénol, un sel métallique d'acide hydroxyméthanesulfinique et la rutine ;
(b) combinaison d'acide isoascorbique ou un sel métallique de celui-ci et d'au moins une espèce sélectionnée à partir du groupe constitué par le 2-mercaptobenzimidazole, le 2,6-di-t-butyl-4-méthylphénol, un sel métallique d'acide hydroxyméthanesulfinique et la rutine ;
(c) combinaison de 2-mercaptobenzimidazole et d'au moins d'une espèce sélectionnée à partir du groupe constitué par le 2,6-di-t-butyl-4-méthylphénol, un sel métallique d'acide hydroxyméthanesulfinique et la rutine ;
(d) combinaison de 2,6-di-t-butyl-4-méthylphénol et d'un sel métallique d'acide hydroxyméthanesulfinique ; et
(e) combinaison d'un sel métallique d'acide hydroxyméthanesulfinique et de rutine.

3. Procédé selon la revendication 2, dans lequel le stabilisant est au moins un type sélectionné à partir des groupes constitués de ce qui suit (a-1) à (c-1) :

(a-1) combinaison d'acide ascorbique, un sel métallique ou un ester de celui-ci et d'au moins une espèce sélectionnée à partir du groupe constitué par l'acide isoascorbique ou un sel métallique de celui-ci, un sel métallique de métabisulfite, le 2,6-di-t-butyl-4-méthylphénol et un sel métallique d'acide hydroxyméthanesulfinique ;
(b-1) combinaison d'acide isoascorbique ou un sel métallique de celui-ci et d'au moins une espèce sélectionnée à partir du groupe constitué par un sel métallique d'acide hydroxyméthanesulfinique et le 2-mercaptobenzimidazole ; et
(c-1) combinaison de 2-mercaptobenzimidazole et d'au moins d'une espèce sélectionnée à partir du groupe constitué par le 2,6-di-t-butyl-4-méthylphénol et un sel métallique d'acide hydroxyméthanesulfinique.

4. Procédé selon l'une quelconque des revendications 1 à 3, qui comprend :

une étape de préparation d'un mélange comprenant l'adhésif sensible à la pression, le donépézil et le stabilisant ; et
une étape de formation de film de la couche adhésive sensible à la pression en appliquant le mélange à une surface du support.

**REFERENCES CITED IN THE DESCRIPTION**

**Patent documents cited in the description**

- JP H11315016 B
- WO 2003032960 A
- WO 2006082728 A
- JP 2000136134 A
- JP 3124069 B
- JP H11047233 B
- JP 2006523637 W
- JP 2003530422 W
- JP 2006523637 PCT
- JP 2003530422 PCT